# EUROPÄISCHE PATENTANMELDUNG

(11) **EP 4 483 914 A1**
(43) Veröffentlichungstag der Anmeldung: **01.01.2025**
(21) Anmeldenummer: 24184676.5
(22) Anmeldetag: 26.06.2024
(51) Int. Cl.: A61M 1/14, A61M 39/24, F16K 37/00

(54) **VORRICHTUNG ZUR EXTRAKORPORALEN BLUTBEHANDLUNG**

(30) Priorität: 29.06.2023 DE 102023117212
(71) Anmelder: B. Braun Avitum AG, 34212 Melsungen (DE)
(72) Erfinder: Knierim, Michael, 37242 Bad Sooden-Allendorf (DE)
(74) Vertreter: Patentanwälte Ruff, Wilhelm, Beier, Dauster & Partner mbB

(57) **Zusammenfassung**

2.1 Eine derartige Vorrichtung zur extrakorporalen Blutbehandlung, aufweisend eine fluidführende Komponente, durch welche ein Fluidleitungspfad erstreckt ist, entlang welchem die fluidführende Komponente im Betrieb der Vorrichtung von einer Flüssigkeit durchströmt ist, ein Stellelement, welches entlang einer in der fluidführenden Komponente erstreckten Bewegungsbahn zwischen einer ersten Stellung und einer zweiten Stellung innerhalb der fluidführenden Komponente beweglich ist, und eine Sensoreinrichtung, welche zum Erfassen der ersten Stellung und/oder der zweiten Stellung des Stellelements eingerichtet ist, ist bekannt.
2.2 Erfindungsgemäß weist die Sensoreinrichtung eine Lichtschranke mit einer Lichtquelle auf, wobei die Lichtquelle an einem Wandabschnitt der fluidführenden Komponente angeordnet und zum Aussenden eines Lichtstrahls eingerichtet ist, wobei der Lichtstrahl die in der fluidführenden Komponente erstreckte Bewegungsbahn des Stellelements kreuzt, und wobei der Lichtstrahl in der ersten Stellung des Stellelements ununterbrochen und in der zweiten Stellung des Stellelements mittels des Stellelements unterbrochen ist.
2.3 Verwendung bei einer extrakorporalen Blutbehandlung

## Beschreibung

Die Erfindung betrifft eine Vorrichtung zur extrakorporalen Blutbehandlung, aufweisend eine fluidführende Komponente, durch welche ein Fluidleitungspfad erstreckt ist, entlang welchem die fluidführende Komponente im Betrieb der Vorrichtung von einer Flüssigkeit durchströmt ist, ein Stellelement, welches entlang einer in der fluidführenden Komponente erstreckten Bewegungsbahn zwischen einer ersten Stellung und einer zweiten Stellung innerhalb der fluidführenden Komponente beweglich ist, und eine Sensoreinrichtung, welche zum Erfassen der ersten Stellung und/oder der zweiten Stellung des Stellelements eingerichtet ist.

Eine derartige Vorrichtung ist im Bereich der Medizintechnik allgemein bekannt und zur Behandlung von Patienten mit beeinträchtigter Nierenfunktion vorgesehen. Bei der bekannten Vorrichtung weist die Sensoreinrichtung einen Reed-Sensor zum Erfassen der Stellung des beweglichen Stellelements auf. In Abhängigkeit der erfassten Stellung kann beispielsweise eine Überwachung oder eine Steuerung des Betriebs der Vorrichtung stattfinden. Der Reed-Sensor ist abseits der fluidführenden Komponente angeordnet und erfasst die Stellung des Stellelements lediglich mittelbar. Zu diesem Zweck ist das im Inneren der fluidführenden Komponente (interne) Stellelement bei der bekannten Vorrichtung mit einem außerhalb der fluidführenden Komponente angeordneten (externen) Bauteil mechanisch wirkverbunden. Das besagte externe Bauteil bewegt sich in Abhängigkeit der Bewegung des Stellelements. Die mechanische Wirkverbindung zwischen dem internen Stellelement und dem externen Bauteil erfordert eine zusätzliche fluiddichte Dichtstelle an der fluidführenden Komponente. Zudem hat es sich gezeigt, dass der üblicherweise verwendete Reed-Sensor prinzipbedingt nicht unter allen Betriebsbedingungen der Vorrichtung ausreichend zuverlässig arbeitet.

Ferner ist aus der EP 3 416 891 B1 eine Anlage zur Herstellung einer medizinischen Zubereitung bekannt. Die bekannte Anlage dient einem Befüllen von Infusionsbeuteln oder Spritzen zur parenteralen Ernährung. Die Anlage weist einen Ventilblock mit mehreren Ventilen auf. Zudem ist eine Sensoreinheit zur Überwachung der jeweiligen Ventilstellungen vorhanden. Die Sensoreinheit umfasst eine Mehrzahl von Lichtschranken, in welche in Abhängigkeit der Ventilstellung ein Finger greift. Der Finger ist ein außerhalb des Ventilblocks angeordnetes (externes) Bauteil. Die Lichtschranken sind abseits des Ventilblocks angeordnet.

Aufgabe der Erfindung ist es, eine Vorrichtung der eingangs genannten Art bereitzustellen, die ein zuverlässiges Erfassen der Stellung des Stellelements ermöglicht und gleichzeitig einen einfachen Aufbau aufweist.

Diese Aufgabe wird dadurch gelöst, dass die Sensoreinrichtung eine Lichtschranke mit einer Lichtquelle aufweist, wobei die Lichtquelle an einem Wandabschnitt der fluidführenden Komponente angeordnet und zum Aussenden eines Lichtstrahls eingerichtet ist, wobei der Lichtstrahl die in der fluidführenden Komponente erstreckte Bewegungsbahn des Stellelements kreuzt, und wobei der Lichtstrahl in der ersten Stellung des Stellelements ununterbrochen und in der zweiten Stellung des Stellelements mittels des Stellelements unterbrochen ist. Die erfindungsgemäße Lösung ermöglicht ein zuverlässiges Erfassen der Stellung des Stellelements. Gleichzeitig weist die erfindungsgemäße Vorrichtung einen einfachen Aufbau auf. Im Unterschied zu aus dem Stand der Technik bekannten Vorrichtungen zur extrakorporalen Blutbehandlung weist die Sensoreinrichtung der erfindungsgemäßen Vorrichtung eine Lichtschranke mit einer erfindungsgemäß angeordneten/ausgerichteten Lichtquelle auf. Dies ermöglicht eine unmittelbare Erfassung der Stellung des Stellelements innerhalb der fluidführenden Komponente. Zu diesem Zweck ist die Lichtquelle an dem Wandabschnitt der fluidführenden Komponente angeordnet und der Lichtstrahl der Lichtquelle kreuzt die Bewegungsbahn des Stellelements innerhalb der fluidführenden Komponente. Der Lichtstrahl wird dementsprechend unmittelbar von dem (internen) Stellelement unterbrochen. Dies im Unterschied zu der aus dem Stand der Technik bekannten Vorrichtung, bei welcher das Erfassen der Stellung des Stellelements mittelbar an dem abseits der fluidführenden Komponente angeordneten (externen) Bauteil erfolgt. Durch die erfindungsgemäße Lösung kann folglich auf die besagte mechanische Wirkverbindung von intern nach extern und damit einhergehend auf eine zusätzliche fluiddichte Dichtstelle an der fluidführenden Komponente verzichtet werden. Dies trägt maßgeblich zu einem vereinfachten Aufbau der Vorrichtung bei. Der Lichtstrahl der Lichtquelle kreuzt die Bewegungsbahn des Stellelements innerhalb der fluidführenden Komponente. Mit anderen Worten ist der Lichtstrahl wenigstens abschnittsweise innerhalb der fluidführenden Komponente erstreckt. Mit nochmals anderen Worten ist der Lichtstrahl in die fluidführende Komponente hinein gerichtet. Bei einer Ausgestaltung ist der Wandabschnitt zu diesem Zweck für das Licht der Lichtquelle transluzent. Bei einer weiteren Ausgestaltung weist der Wandabschnitt eine Aussparung auf, in welche die Lichtquelle eingesetzt ist, wodurch auf eine transluzente Gestaltung des Wandabschnitts verzichtet werden kann. Denkbar und möglich ist es zudem, dass die Lichtquelle innerhalb der fluidführenden Komponente angeordnet ist, so dass der Wandabschnitt nicht unbedingt transluzent sein oder eine Aussparung aufweisen muss. Die Vorrichtung ist vorzugsweise ein Dialysegerät zur Hämodialyse. Die fluidführende Komponente weist bei unterschiedlichen Ausgestaltungen der Erfindung einen unterschiedlichen Aufbau und/oder eine unterschiedliche Funktion auf. Bei einer Ausgestaltung ist die fluidführende Komponente ein Ventil, insbesondere ein Rückschlagventil, wobei das Stellelement in diesem Fall bevorzugt ein Ventilkörper zum Öffnen und Schließen des Ventils ist. Bei einer weiteren Ausgestaltung der Erfindung ist die fluidführende Komponente ein Fluidkonnektor zur fluidleitenden Verbindung mit einer Schlauchleitung, einer Rohrleitung oder dergleichen, wobei das Stellelement in diesem Fall bevorzugt ein Verschlusselement zum Öffnen und Schließen des Fluidkonnektors ist. Bei einer weiteren Ausgestaltung ist die fluidführende Komponente eine Schlauchleitung, eine Rohrleitung oder dergleichen, wobei das Stellelement in diesem Fall vorzugsweise ein innerhalb der Schlauch- oder Rohrleitung beweglicher Ansaugstab, Köcher oder dergleichen ist. Der durch die fluidführende Komponente erstreckte Fluidleitungspfad ist bei einer Ausgestaltung der Erfindung ein Abschnitt eines Fluidsystems, insbesondere eines Behandlungsflüssigkeitspfads, der Vorrichtung, entlang welchem im Betrieb der Vorrichtung ein Fluid strömt. Bei dem Fluid kann es sich insbesondere um frische und/oder verbrauchte Behandlungsflüssigkeit handeln. Bei der Behandlungsflüssigkeit kann es sich beispielsweise um Dialysierflüssigkeit handeln. Die fluidführende Komponente wird in diesem Fall im Betrieb der Vorrichtung von der besagten Behandlungsflüssigkeit durchströmt. Bei einer weiteren Ausgestaltung der Erfindung ist der Fluidleitungspfad ein Abschnitt eines extrakorporalen Blutkreislaufs, entlang welchem zu behandelndes Blut strömt. Bei einer weiteren Ausgestaltung der Erfindung ist der Fluidleitungspfad ein Abschnitt, der weder dem Behandlungsflüssigkeitspfad noch dem extrakorporalen Blutkreislauf zugeordnet ist. Die Lichtschranke weist vorzugsweise eine als solche bekannte Bauart auf und kann beispielsweise eine Einweglichtschranke (Gabellichtschranke), eine Reflexionslichtschranke oder ein Reflexionslichttaster sein. Dabei versteht sich, dass zusätzlich zu der besagten Lichtquelle ein Lichtdetektor zum Empfangen des Lichtstrahls der Lichtquelle vorhanden ist. Je nach Bauform der Lichtschranke kann der Lichtdetektor in Bezug auf die Lichtquelle unterschiedlich angeordnet sein. Bei einer bevorzugten Ausführungsform ist die Lichtschranke in unterbrochenem Zustand des Lichtstrahls ausgelöst oder betätigt. In ununterbrochenem Zustand des Lichtstrahls ist die Lichtschranke nicht ausgelöst oder nicht betätigt. Es ist selbstverständlich auch eine hierzu umgekehrte Zuordnung von ausgelöstem und nicht ausgelöstem Zustand der Lichtschranke denkbar und möglich. In unterbrochenem Zustand des Lichtstrahls kann keine Detektion mittels des Lichtdetektors erfolgen. In ununterbrochenem Zustand des Lichtstrahls kann eine Detektion mittels des Lichtdetektors erfolgen.

In Ausgestaltung der Erfindung weist der Wandabschnitt für den Lichtstrahl der Lichtquelle transluzente Eigenschaften auf, wobei die Lichtquelle auf einer Außenseite des Wandabschnitts angeordnet ist, und wobei die Bewegungsbahn des Stellelements entlang einer der Außenseite gegenüberliegenden Innenseite des Wandabschnitts erstreckt ist. Die Außenseite des Wandabschnitts ist einer Umgebung zugewandt. Die der Außenseite gegenüberliegende Innenseite des Wandabschnitts ist dem Stellelement, dessen Bewegungsbahn und/oder dem Fluidleitungspfad zugewandt. Im Betrieb der Vorrichtung strömt die Flüssigkeit vorzugsweise entlang der Innenseite des Wandabschnitts. Bei dieser Ausgestaltung der Erfindung ist der Wandabschnitt vorzugsweise aus einem Kunststoffmaterial mit transluzenten Eigenschaften gefertigt. Alternativ ist eine Fertigung aus Glas denkbar und möglich.

In weiterer Ausgestaltung der Erfindung weist der Wandabschnitt eine Apertur auf, welche quer zu der Bewegungsbahn zwischen einer Innenseite, entlang welcher die Bewegungsbahn des Stellelements erstreckt ist, und einer der Innenseite gegenüberliegenden Außenseite des Wandabschnitts durchgängig erstreckt ist, wobei die Lichtquelle in die Apertur eingesetzt ist. Die Lichtquelle ist vorzugsweise fluiddicht in die Apertur eingesetzt, beispielsweise mittels einer fluiddichten Fügeverbindung und/oder einem Dichtungsbauteil. Als fluiddichte Fügeverbindung kommt insbesondere eine Klebeverbindung infrage. Hinsichtlich der Ausrichtung der Innenseite und der Außenseite gilt, mutatis mutandis, das zu der vorhergehenden Ausgestaltung der Erfindung Gesagte.

In weiterer Ausgestaltung der Erfindung sind die Bewegungsbahn und der Fluidleitungspfad im Bereich des Wandabschnitts parallel. Bei dieser Ausgestaltung der Erfindung strömt die Flüssigkeit im Betrieb der Vorrichtung unmittelbar entlang der Innenseite des Wandabschnitts. Dabei ist die Strömung parallel zu der Bewegungsbahn des Stellelements. Diese Ausgestaltung geht von der Überlegung aus, dass - jedenfalls in der Theorie - eine Ablagerung von Stoffen an der Innenseite des Wandabschnitts grundsätzlich denkbar ist. Solche Ablagerungen können zu einer Beeinträchtigung des Lichtstrahls der Lichtquelle und damit zu einer beeinträchtigten Funktion der Lichtschranke führen. Durch die parallele Ausrichtung von Bewegungsbahn und Fluidleitungspfad im Bereich des Wandabschnitts kann solchen Ablagerungen vorgebeugt werden.

In weiterer Ausgestaltung der Erfindung weist der Wandabschnitt eine Lagerfläche auf, an welcher das Stellelement entlang der Bewegungsbahn gleitbeweglich gelagert ist. Die Lagerfläche ist an einer/der Innenseite des Wandabschnitts angeordnet. Das Stellelement bewegt sich entlang der Lagerfläche zwischen der ersten und der zweiten Stellung. Die Lagerfläche dient einer gleitbeweglichen Führung des Stellelements. Bei dieser Ausgestaltung weist der Wandabschnitt eine vorteilhafte Mehrfachfunktion auf. Zum einen dient er der Anordnung der Lichtquelle. Zum anderen dient er der Lagerung des Stellelements. Hierdurch kann eine besonders kompakte Bauweise der Vorrichtung erreicht werden.

In weiterer Ausgestaltung der Erfindung ist der Wandabschnitt ein Abschnitt eines Gehäuses der fluidführenden Komponente, wobei der Fluidleitungspfad wenigstens abschnittsweise durch das Gehäuse erstreckt ist und das Stellelement in dem Gehäuse beweglich gelagert ist. Bei dieser Ausgestaltung der Erfindung kann der Wandabschnitt auch als Gehäusewandabschnitt bezeichnet werden. Sofern der Gehäusewandabschnitt transluzente Eigenschaften gemäß einer der vorhergehenden Ausgestaltungen aufweist, ist das Gehäuse vorzugsweise aus unterschiedlichen Materialien gefertigt, wobei der besagte Gehäusewandabschnitt dementsprechend aus einem transluzenten Material besteht. Sofern stattdessen gemäß einer der vorhergehenden Ausgestaltungen eine Apertur zur Aufnahme der Lichtquelle vorhanden ist, kann diese als Gehäuseöffnung oder Gehäusebohrung bezeichnet werden.

In weiterer Ausgestaltung der Erfindung ist die fluidführende Komponente ein Ventil, insbesondere ein Rückschlagventil, und das Stellelement ist ein Ventilkörper. Ein solches Ventil kann an unterschiedlichen Stellen eines Fluidsystems der Vorrichtung angeordnet sein und unterschiedliche Funktionen aufweisen. Das Ventil dient einer Steuerung der Strömung entlang des Fluidleitungspfads.

In weiterer Ausgestaltung der Erfindung ist der Wandabschnitt ein Abschnitt eines Ventilgehäuses des Ventils, wobei das Ventilgehäuse einen Einlass zum Einleiten der Flüssigkeit und einen entlang des Fluidleitungspfads fluidleitend mit dem Einlass verbundenen Auslass zum Ausleiten der Flüssigkeit aufweist, und wobei die Lichtquelle entlang des Fluidleitungspfads zwischen dem Einlass und dem Auslass an dem Wandabschnitt des Ventilgehäuses angeordnet ist.

Die Erfindung betrifft zudem eine Anordnung, aufweisend eine fluidführende Komponente, durch welche ein Fluidleitungspfad erstreckt ist, entlang welchem die fluidführende Komponente von einer Flüssigkeit durchströmbar ist, ein Stellelement, welches entlang einer in der fluidführenden Komponente erstreckten Bewegungsbahn zwischen einer ersten Stellung und einer zweiten Stellung innerhalb der fluidführenden Komponente beweglich ist, und eine Sensoreinrichtung, welche zum Erfassen der ersten Stellung und/oder der zweiten Stellung des Stellelements eingerichtet ist, wobei die Sensoreinrichtung eine Lichtschranke mit einer Lichtquelle aufweist, wobei die Lichtquelle an einem Wandabschnitt der fluidführenden Komponente angeordnet und zum Aussenden eines Lichtstrahls eingerichtet ist, wobei der Lichtstrahl die in der fluidführenden Komponente erstreckte Bewegungsbahn des Stellelements kreuzt, und wobei der Lichtstrahl in der ersten Stellung des Stellelements ununterbrochen und in der zweiten Stellung des Stellelements mittels des Stellelements unterbrochen ist. Die Anordnung eignet sich in bevorzugter Weise für eine Vorrichtung zur extrakorporalen Blutbehandlung. Die Anordnung kann aber auch an anderen medizinischen Vorrichtungen verwendet werden und ist nicht auf eine Verwendung an Vorrichtungen zur extrakorporalen Bluthandlung eingeschränkt.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Perspektivansicht eine Ausführungsform einer erfindungsgemäßen Vorrichtung zur extrakorporalen Blutbehandlung,
- Fig. 2: die Vorrichtung nach Fig. 1 in einer weiteren perspektivischen Ansicht, wobei Funktionsarme der Vorrichtung einen geöffneten Zustand einnehmen,
- Fig. 3: eine vergrößerte perspektivische Detailansicht der Vorrichtung nach den Fig. 1 und 2 im Bereich der Funktionsarme und mit Blickrichtung auf zwei fluidführende Komponenten der Vorrichtung,
- Fig. 4: eine der fluidführenden Komponenten der Vorrichtung nach den Fig. 1 und 3 in einem perspektivischen Längsschnitt,
- Fig. 5, 6: Längsschnittansichten der fluidführenden Komponente nach Fig. 4, wobei ein bewegliches Stellelement der fluidführenden Komponente eine erste Stellung (Fig. 5) und eine zweite Stellung einnimmt (Fig. 6),
- Fig. 7: eine erfindungsgemäße Variante der fluidführenden Komponente nach den Fig. 4 bis 6 in einer perspektivischen Längsschnittansicht,
- Fig. 8, 9: die Variante der fluidführenden Komponente nach Fig. 7 in weiteren Längsschnittansichten, wobei das Stellelement eine erste Stellung (Fig. 8) und eine zweite Stellung einnimmt (Fig. 9),
- Fig. 10, 11: Längsschnittansichten einer nicht erfindungsgemäßen Variante der fluidführenden Komponente in einer ersten Stellung des Stellelements (Fig. 10) und einer zweiten Stellung des Stellelements (Fig. 11), und
- Fig. 12, 13: Längsschnittansichten einer weiteren nicht erfindungsgemäßen Variante der fluidführenden Komponente in einer ersten Stellung des Stellelements (Fig. 12) und einer zweiten Stellung des Stellelements (Fig. 13).

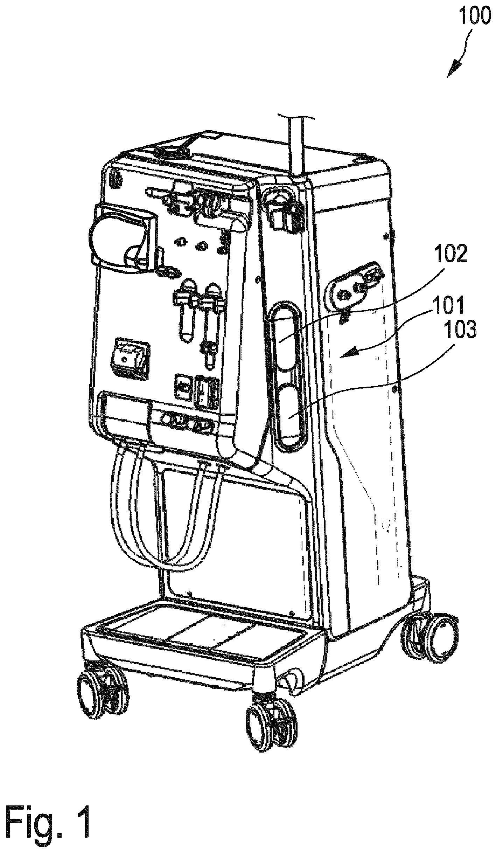

Gemäß den Fig. 1 bis 3 ist eine Vorrichtung 100 zur extrakorporalen Blutbehandlung vorgesehen. Bei der gezeigten Ausführungsform ist die Vorrichtung 100 ein Dialysegerät zur Hämodialyse.

Der prinzipielle Aufbau und das Funktionsprinzip der Vorrichtung 100 sind dem Fachmann bekannt. Die Blutbehandlung, im vorliegenden Fall die Dialyse, erfolgt über einen Diffusionsprozess innerhalb eines Dialysators. Der Dialysator ist in den Figuren nicht gezeigt.

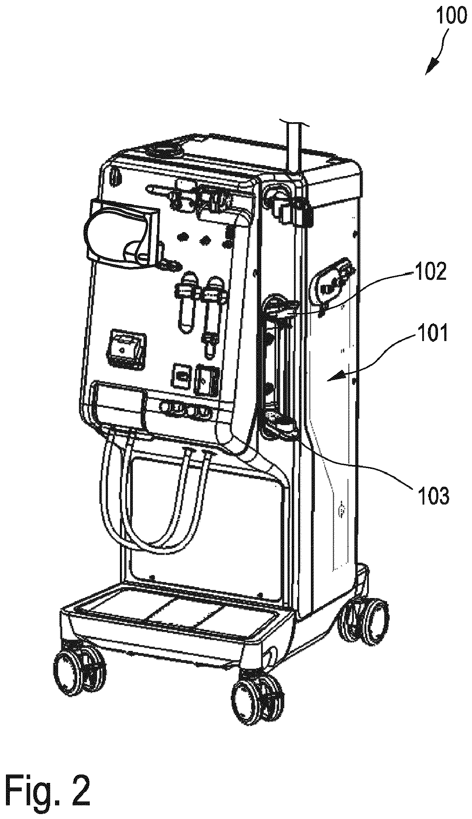

Die Vorrichtung 100 weist zudem einem Funktionsbereich 101 mit zwei Funktionsarmen 102, 103 auf. Die Funktionsarme 102, 103 sind in der in Fig. 1 gezeigten Konfiguration eingeklappt, so dass der Funktionsbereich 101 verdeckt ist. In der in Fig. 2 gezeigten Konfiguration sind die Funktionsarme 102, 103 aufgeklappt. In dem aufgeklappten Zustand ist der Funktionsbereich 101 freigegeben, so dass eine Funktionseinheit zwischen den Funktionsarmen 102, 103 aufnehmbar ist.

Bei der gezeigten Ausführungsform handelt es sich bei der besagten Funktionseinheit um eine Bicarbonatkartusche (BIC-Kartusche). Die Bicarbonatkartusche ist in den Figuren nicht gezeigt. In dem aufgeklappten Zustand der Funktionsarme 102, 103 ist die Bicarbonatkartusche an dem Funktionsbereich 101 zwischen den beiden Funktionsarmen 102, 103 aufnehmbar.

Die Bicarbonatkartusche ist in einem zwischen den Funktionsarmen 102, 103 aufgenommenen Zustand fluidleitend mit einem Fluidsystem der Vorrichtung 100 verbunden. Das Fluidsystem umfasst insbesondere einen Dialysierflüssigkeitskreislauf oder auch -pfad und einen extrakorporalen Blutkreislauf.

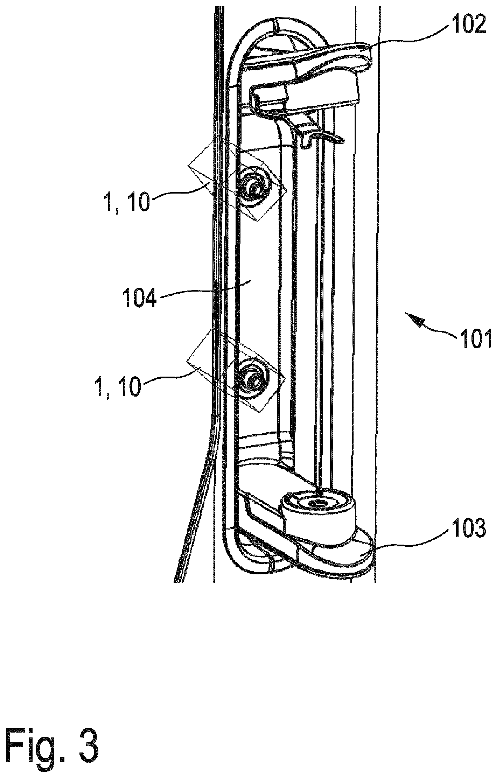

Die fluidleitende Verbindung der Bicarbonatkartusche mit dem Fluidsystem erfolgt vorliegend über fluidführende Komponenten 1 (siehe Fig. 3). Die fluidführenden Komponenten 1 sind in eingeklapptem Zustand der Funktionsarme 102, 103 (siehe Fig. 1) verdeckt und in ausgeklapptem Zustand der Funktionsarme (Fig. 2, 3) freigegeben und insoweit zugänglich. Die fluidführenden Komponenten 1 sind vorliegend an einem Panelabschnitt 104 eines Seitenpanels (ohne Bezugszeichen) der Vorrichtung 100 angebracht. Die fluidführenden Komponenten 1 ragen einends durch nicht näher bezeichnete Öffnungen aus dem Panelabschnitt 104 heraus und sind in wesentlichen Teilen hinter dem Panelabschnitt 104 angeordnet. Ein hinter dem Panelabschnitt 104 liegender Bauraum der fluidführenden Komponenten 1 ist in Fig. 3 jeweils quaderförmig schematisch eingezeichnet. Dabei ist die Art der Anordnung und Anbringung der fluidführenden Komponenten 1 nicht wesentlich für die vorliegende Erfindung. Nähere diesbezügliche Erläuterungen sind daher entbehrlich.

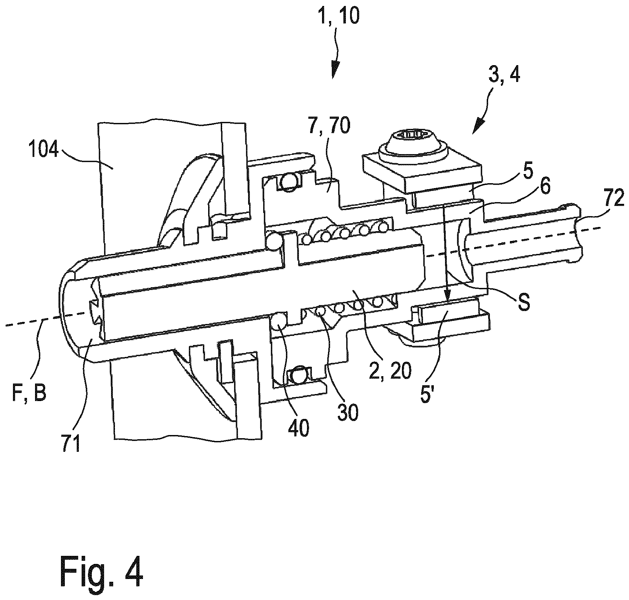

Nachfolgend wird unter Bezugnahme auf die Fig. 4 bis 6 der spezifische Aufbau und die Funktion der fluidführenden Komponenten 1. Da die in Fig. 3 gezeigten fluidführenden Komponenten 1 einen identischen Aufbau aufweisen, wird dabei lediglich auf eine/die fluidführende Komponente 1 Bezug genommen.

Durch die fluidführende Komponente 1 ist ein Fluidleitungspfad F erstreckt. Der Fluidleitungspfad F bildet einem Abschnitt des Fluidsystems der Vorrichtung 100. Im Betrieb der Vorrichtung 100 wird die fluidführende Komponente 1 entlang des Fluidleitungspfads F von Flüssigkeit durchströmt.

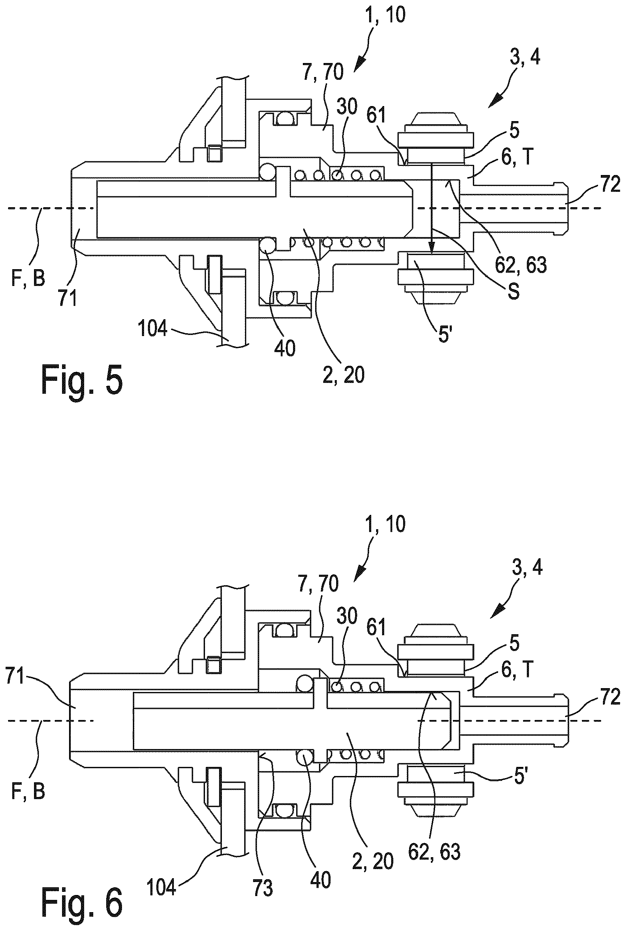

Weiter ist ein Stellelement 2 vorhanden. Das Stellelement 2 ist entlang einer in der fluidführenden Komponente 1 erstreckten Bewegungsbahn B zwischen einer ersten Stellung (Fig. 4, 5) und einer zweiten Stellung (Fig. 6) relativ zu der fluidführenden Komponente 1 beweglich.

Bei der gezeigten Ausführungsform ist das Stellelement 2 translatorisch zwischen der ersten Stellung und der zweiten Stellung verlagerbar. Bei einer in den Figuren nicht gezeigten Ausführungsform ist das Stellelement alternativ oder zusätzlich rotatorisch beweglich. Denkbar und möglich ist zudem eine Schwenkbeweglichkeit des Stellelements.

Weiter ist eine Sensoreinrichtung 3 vorhanden und zum Erfassen der Stellung des Stellelements 2 eingerichtet.

Die Sensoreinrichtung 3 weist eine Lichtschranke 4 mit einer Lichtquelle 5 auf. Die Lichtquelle 5 ist an einem Wandabschnitt 6 der fluidführenden Komponente 1 angeordnet und zum Aussenden eines Lichtstrahls S eingerichtet. Wie insbesondere in den Fig. 4 und 5 gezeigt ist, kreuzt der Lichtstrahl S die in der fluidführenden Komponente 1 erstreckte Bewegungsbahn B des Stellelements 2. In der ersten Stellung des Stellelements 2 (Fig. 4, 5) ist der besagte Lichtstrahl S ununterbrochen. In der zweiten Stellung (Fig. 6) unterbricht das Stellelement 2 den Lichtstrahl S.

Bei der gezeigten Ausführungsform ist die Lichtschranke 4 in unterbrochenem Zustand des Lichtstrahls S ausgelöst oder betätigt. In ununterbrochenem Zustand des Lichtstrahls S ist die Lichtschranke 4 nicht ausgelöst oder nicht betätigt. Es ist selbstverständlich auch eine hierzu umgekehrte Zuordnung von ausgelöstem und nicht ausgelöstem Zustand der Lichtschranke 4 denkbar und möglich.

Weiter ist in den Fig. 4 bis 6 gezeigt, dass die Lichtschranke 4, insbesondere deren Lichtquelle 5, unmittelbar an dem Wandabschnitt 6 der fluidführenden Komponente 1 angeordnet ist. Der Lichtstrahl S, der auch als Lichtsignal bezeichnet werden kann, ist dabei in das Innere der fluidführenden Komponente 1 hinein gerichtet, so dass er von dem dort beweglichen Stellelement 2 unterbrochen werden kann. Das Erfassen der Stellung des Stellelements 2 erfolgt insoweit unmittelbar (direkt) an dem Stellelement 2 und im Inneren (intern) der fluidführenden Komponente 1, so dass auch von einer direkten und internen Erfassung der Stellung gesprochen werden kann.

Die in den Fig. 4 bis 6 exemplarisch gezeigte Lichtschranke 4 ist als Platzhalter für unterschiedliche Gestaltungen zu verstehen. Grundsätzlich sind unterschiedlichste Bauarten der Lichtschranke denkbar und möglich. Beispielsweise kann die Lichtschranke als Einweglichtschranke (Gabellichtschranke), Reflexionslichtschranke oder Reflexionslichttaster gestaltet sein. Die spezifische Gestaltung der Lichtschranke ist im Hinblick auf die Erfindung nicht wesentlich. Wesentlich ist vielmehr, dass die Lichtquelle an einem Wandabschnitt der fluidführenden Komponente angeordnet und zum Aussenden eines Lichtstrahls eingerichtet ist, wobei der Lichtstrahl die in der fluidführenden Komponente erstreckte Bewegungsbahn des Stellelements kreuzt, und wobei der Lichtstrahl in der ersten Stellung des Stellelements ununterbrochen und in der zweiten Stellung des Stellelements mittels des Stellelements unterbrochen ist. Dabei versteht es sich, dass die Lichtschranke 4 zusätzlich zu der Lichtquelle 5 einen Lichtdetektor zum Detektieren des Lichtstrahls S aufweist. Allerdings sind die Art und Anordnung des Lichtdetektors bei unterschiedlichen Bauweisen unterschiedlich. Auf die in den Fig. 4 bis 6 gezeigte spezifische Gestaltung wird nachfolgend noch näher eingegangen.

Bei der in den Fig. 4 bis 6 gezeigten Ausführungsform weist der Wandabschnitt 6 transluzente Eigenschaften T auf. Mit anderen Worten ausgedrückt, ist der Wandabschnitt 6 für den Lichtstrahl S der Lichtquelle 5 durchgängig. Weiter ist die Lichtquelle 5 bei der gezeigten Ausführungsform auf einer Außenseite 61 des Wandabschnitts 6 angeordnet. Die Bewegungsbahn B des Stellelements 2 ist entlang einer Innenseite 62 des Wandabschnitts erstreckt. Die Außenseite 61 und die Innenseite 62 liegen einander quer, vorliegend orthogonal, zu der Bewegungsbahn B gegenüber. Dabei ist die Außenseite 61 einer nicht näher bezeichneten Umgebung zugewandt. Die Innenseite 62 ist dem Stellelement 2 und dessen Bewegungsbahn B zugewandt. Durch die transluzenten Eigenschaften T des Wandabschnitts 6 kann der Lichtstrahl S der Lichtquelle 5 ausgehend von der Außenseite 61 durch den Wandabschnitt 6 hindurch bis auf die Innenseite 62 und von dort weiter in das Innere der fluidführenden Komponente 1 gelangen. Der Lichtstrahl S ist folglich durch den transluzenten Wandabschnitt 6 in das Innere der fluidführenden Komponente 1 gerichtet.

Bei der gezeigten Ausführungsform weist die Lichtschranke 4 einen Lichtdetektor 5' auf. Der Lichtdetektor 5` liegt der Lichtquelle 5 entlang des Lichtstrahls S und/oder quer, vorzugsweise orthogonal, zu der Bewegungsbahn B gegenüber. Der Lichtdetektor 5' ist ebenfalls an einer Außenseite des Wandabschnitts 6 angeordnet. Dabei ist der Lichtdetektor 5' vorliegend um 180° um die Bewegungsbahn B des Stellelements 2 zu der Lichtquelle 5 versetzt angeordnet. In der zweiten Stellung (Fig. 6) ist das Stellelement 2 in Richtung des Lichtstrahls S - und damit radial zu der Bewegungsbahn B - zwischen der Lichtquelle 5 und dem Lichtdetektor 5' angeordnet; der Lichtstrahl S ist in der zweiten Stellung unterbrochen und somit mittels des Lichtdetektors 5' nicht detektierbar.

Bei der gezeigten Ausführungsform ist der transluzente Wandabschnitt 6 aus einem nicht näher bezeichneten transluzenten Kunststoffmaterial gefertigt. Alternativ denkbar und möglich ist eine Fertigung des Wandabschnitts 6 aus Glas. Entscheidend ist, dass der Wandabschnitt 6 für den Lichtstrahl S der Lichtquelle 5 transluzent ist. Je nach Wellenlänge des Lichtstrahls S sind die transluzenten Eigenschaften T bei unterschiedlichen Ausgestaltungen unterschiedlich. Insbesondere ist es nicht erforderlich, dass der Wandabschnitt 6 transparent, das heißt durchsichtig, und folglich für sichtbares Licht durchgängig ist.

Bei der in den Fig. 4 bis 6 gezeigten Ausführungsform weist die Innenseite 62 des Wandabschnitts 6 zudem eine Lagerfläche 63 auf. Das Stellelement 2 ist bei einer Bewegung zwischen seiner ersten Stellung und seiner zweiten Stellung entlang der Bewegungsbahn B gleitbeweglich an der Lagerfläche 63 gelagert.

Der Wandabschnitt 6 weist folglich eine vorteilhafte Mehrfachfunktion auf. Denn zum einen dient der Wandabschnitt 6 der Anbringung der Lichtschranke 4. Zum anderen fungiert der Wandabschnitt 6, genauer dessen Innenseite 62, als Lagerfläche 63 für das Stellelement 2.

Bei der in den Fig. 4 bis 6 gezeigten Ausführungsform ist der Wandabschnitt 6 ein Abschnitt eines Gehäuses 7 der fluidführenden Komponente 1. Der Fluidleitungspfad F ist wenigstens abschnittsweise durch das Gehäuse 7 erstreckt. Das Stellelement 2 ist beweglich in dem Gehäuse 7 gelagert. Die Lichtschranke 4 ist folglich unmittelbar an dem Gehäuse 7 angebracht. Das Gehäuse 7 ist form- und/oder kraftschlüssig an dem Panelabschnitt 104 befestigt, wobei die Art und Weise der Befestigung für die vorliegende Erfindung nicht wesentlich sind.

Bei der in den Fig. 4 bis 6 gezeigten Ausführungsform sind der Fluidleitungspfad F und die Bewegungsbahn B wenigstens im Bereich des Wandabschnitts 6 parallel erstreckt. Im Betrieb der Vorrichtung 100 ist die Innenseite 62 des Wandabschnitts 6 von Flüssigkeit benetzt. Die Flüssigkeit strömt hierbei entlang des Wandabschnitts 6, genauer dessen Innenseite 62. Der Lichtstrahl S ist folglich in die entlang des Flüssigkeitspfads F strömende Flüssigkeit gerichtet.

Bei der in den Fig. 4 bis 6 gezeigten Ausführungsform ist die fluidführende Komponente 1 ein Ventil 10. Das Ventil 10 ist vorliegend ein Rückschlagventil. Das Stellelement 2 ist ein Ventilkörper 20 des Ventils 10. In der ersten Stellung (Fig. 4, 5) des Ventilkörpers 20 ist das Ventil 10 geschlossen. In der zweiten Stellung (Fig. 6) ist das Ventil 10 geöffnet. Dabei ist der Ventilkörper 20 vorliegend mittels einer Ventilfeder 30 in Richtung seiner ersten Stellung vorgespannt. In der ersten Stellung dichtet der Ventilkörper 20 den Fluidleitungspfad F fluiddicht ab. Hierzu wirkt der Ventilkörper 20 mit einem Ventilsitz 73 zusammen. Der Ventilsitz 73 ist ein Abschnitt des Gehäuses 7. Das Gehäuse 7 kann bei der gezeigten Ausführungsform - aufgrund der Gestaltung der fluidführenden Komponente 1 als Ventil 10 - auch als Ventilgehäuse 70 bezeichnet werden kann. In der ersten Stellung dichtet der Ventilkörper 20 den Ventilsitz 73 über ein zusätzliches Dichtelement 40 fluiddicht ab.

Vorliegend weist das Ventilgehäuse 70 einen Einlass 71 und einen Auslass 72 auf. Der Einlass 71 und der Auslass 72 sind über den Fluidleitungspfad F fluidleitend miteinander verbunden. Die besagte fluidleitende Verbindung besteht in der zweiten Stellung des Ventilkörpers 20. In der ersten Stellung (Fig. 4, 5) ist das Ventil 10 geschlossen, die fluidleitende Verbindung zwischen Einlass 71 und Auslass 72 ist aufgehoben.

Die Lichtschranke 4, insbesondere deren Lichtquelle 5, ist entlang des Fluidleitungspfads F zwischen dem Einlass 71 und dem Auslass 72 an dem Wandabschnitt 6 des Ventilgehäuses 70 angebracht.

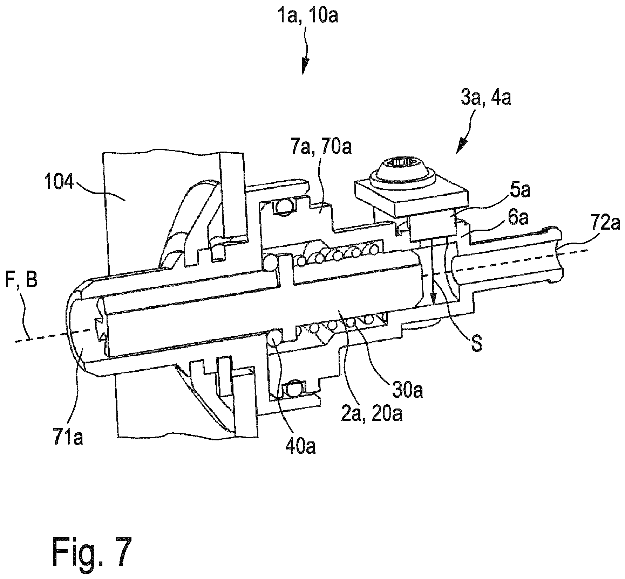

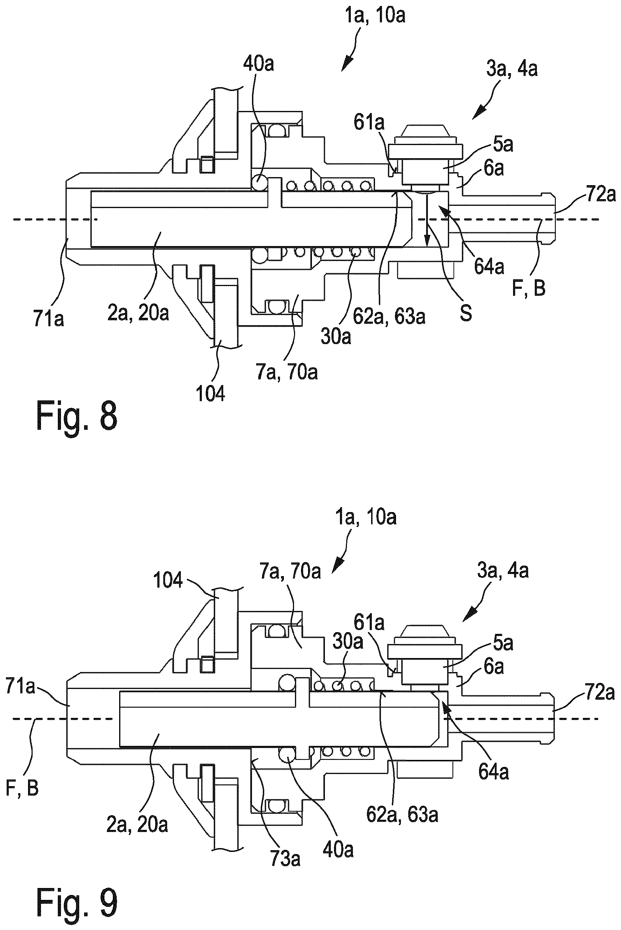

In den Fig. 7 bis 9 ist eine fluidführende Komponente 1a als Variante der fluidführenden Komponente 1 nach den Fig. 4 bis 6 gezeigt. Die fluidführende Komponente 1a ist weitgehend identisch mit der fluidführenden Komponente 1 nach den Fig. 4 bis 6. Nachfolgend werden lediglich wesentliche Unterschiede der fluidführenden Komponente 1a nach den Fig. 7 bis 9 gegenüber der fluidführenden Komponente 1 nach den Fig. 4 bis 6 erläutert. Funktionsgleiche Bauteile und/oder Abschnitte werden nicht gesondert erläutert und sind durch identische Bezugszeichenziffern unter Hinzufügung von Kleinbuchstaben gekennzeichnet.

Bei der fluidführenden Komponente 1a weist der Wandabschnitt 6a eine Apertur 64a auf. Die Apertur 64a ist durchgängig zwischen der Außenseite 61a und der Innenseite 62a des Wandabschnitts 6a axial erstreckt. Die Apertur 64a kann auch als Gehäuseöffnung des Gehäuses 7a, insbesondere des Ventilgehäuses 70a, bezeichnet werden. Die Lichtquelle 5a ist in die Apertur 64a eingesetzt.

Bei dieser Variante kann folglich auf eine transluzente Gestaltung des Wandabschnitts 6a verzichtet werden. Anstelle transluzenter Eigenschaften T ist die besagte Apertur 64a zur Aufnahme der Lichtquelle 5a vorhanden. Die Lichtquelle 5a gibt den Lichtstrahl S folglich unmittelbar an der Innenseite 62a des Wandabschnitts 6a ab. Der Lichtquelle 5a kann dabei in Richtung der Innenseite 62a eine Linse, eine Scheibe oder Sonstiges vorgelagert sein. Die Lichtquelle 5a ist fluiddicht in die Apertur 64a eingesetzt. Die fluiddichte Abdichtung zwischen der Innenseite 62a und der Außenseite 61a kann dadurch erreicht werden, dass die Lichtquelle 5a in die Apertur 64a eingeklebt oder auf sonstige Weise fluiddicht eingesetzt wird. Alternativ oder zusätzlich zu einer fluiddichten Fügeverbindung kann ein O-Ring oder sonstiges Dichtelement vorhanden sein.

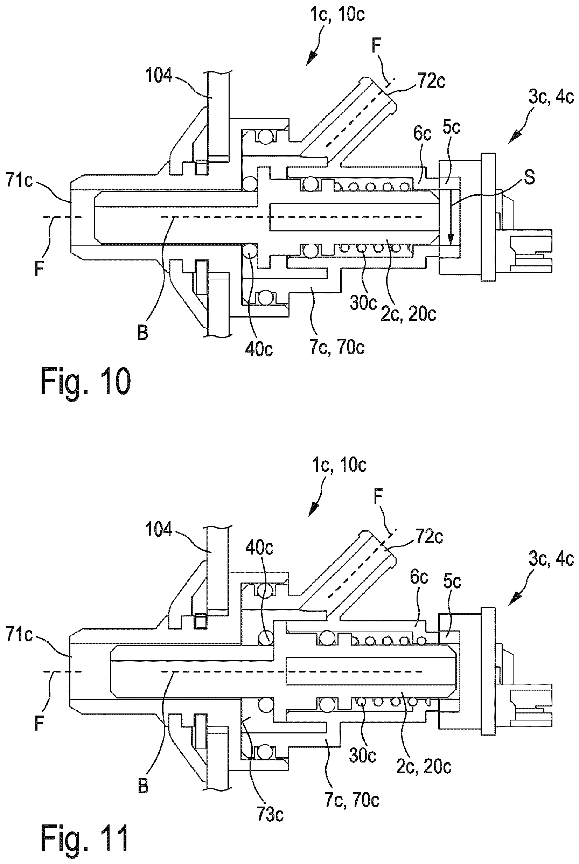

In den Fig. 10 und 11 sowie 12 und 13 sind weitere fluidführende Komponenten 1c bzw. 1d gezeigt. Bei den in den Fig. 10 bis 13 gezeigten fluidführenden Komponenten 1c, 1d handelt es sich um nicht erfindungsgemäße Gestaltungen. Dessen ungeachtet sind die fluidführenden Komponenten 1c, 1d in Teilen identisch mit den erfindungsgemäß gestalteten fluidführenden Komponenten 1, 1a. Übereinstimmende Bauteile und Abschnitte werden nicht gesondert erläutert.

Bei der in den Fig. 10 und 11 gezeigten fluidführenden Komponente 1c ist die Lichtschranke 4c nicht zur "internen" Erfassung der Stellung des Stellelements 2c eingerichtet. Vielmehr wird die Stellung des Stellelements 2c außerhalb der fluidführenden Komponente, im Speziellen das Gehäuse 7c (Ventilgehäuse 70c), erfasst. Das Gehäuse 7c weist folglich eine Durchgangsöffnung (ohne Bezugszeichen) für das Stellelement 2c auf. In der zweiten Stellung (Fig. 11) ist ein Stirnende des Stellelements 2c durch die Durchgangsöffnung aus dem Gehäuse 7c heraus verlagert. Die Bewegungsbahn B des Stellelement ist folglich nicht (vollständig) im inneren der des Gehäuses 7c erstreckt. In dem aus dem Gehäuse 7c herausverlagerten Zustand des Stellelement 2c wird die Lichtschranke 4c durch eine Unterbrechung des Lichtstrahls S ausgelöst. Der Lichtstrahl S wird folglich nicht etwa in die fluidführende Komponente 1c hinein gerichtet. Durch die bei dieser nicht erfindungsgemäßen Variante erforderliche Verlagerung des Stellelements 2c nach außen ist eine zusätzliche Abdichtung des Gehäuses 7c im Bereich der Durchgangsöffnung erforderlich. Zudem kann der Fluidleitungspfad F nicht wie bei den Varianten nach den Fig. 4 bis 9 parallel zu der Bewegungsbahn B geführt werden. Durch die stirnendseitig des Stellelements 2c erforderliche Anordnung der Lichtschranke 4c muss der Fluidleitungspfad F vielmehr abgekröpft werden. Der Auslass 72c ist folglich seitlich des Gehäuses 7c und nicht etwa stirnendseitig angeordnet.

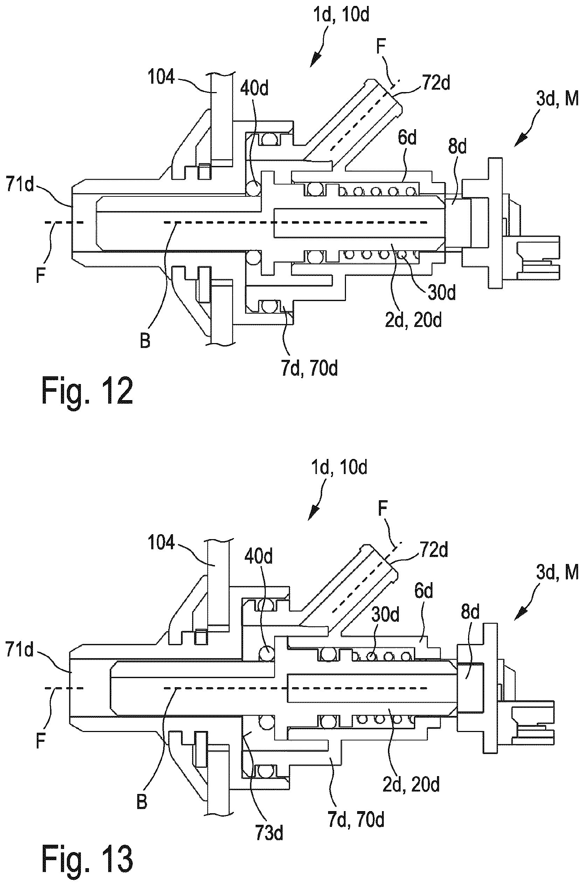

Die nicht erfindungsgemäße fluidführende Komponente 1d nach den Fig. 12 und 13 ist im Wesentlichen identisch zu der fluidführenden Komponente 1c nach den Fig. 10 und 11. Im Unterschied zu dieser weist die Sensoreinrichtung 3d anstelle der Lichtschranke 4c einen Mikroschalter M auf. Der Mikroschalter M ist entsprechend der Lichtschranke 4c endseitig des Stellelements 2d und damit auch des Gehäuses 7d angeordnet. Die Betätigung des Mikroschalters M erfolgt über ein Betätigungselement 8d, das mit einem dem Mikroschalter M zugewandten Stirnende des Stellelements 2d fest verbunden ist.

## Patentansprüche

1. Vorrichtung (100) zur extrakorporalen Blutbehandlung, aufweisend
eine fluidführende Komponente (1, 1a), durch welche ein Fluidleitungspfad (F) erstreckt ist, entlang welchem die fluidführende Komponente (1, 1a) im Betrieb der Vorrichtung (100) von einer Flüssigkeit durchströmt ist,
ein Stellelement (2, 2a), welches entlang einer in der fluidführenden Komponente (1, 1a) erstreckten Bewegungsbahn (B) zwischen einer ersten Stellung und einer zweiten Stellung innerhalb der fluidführenden Komponente (1, 1a) beweglich ist,
und eine Sensoreinrichtung (3, 3a), welche zum Erfassen der ersten Stellung und/oder der zweiten Stellung des Stellelements (2, 2a) eingerichtet ist,
**dadurch gekennzeichnet, dass** die Sensoreinrichtung (3, 3a) eine Lichtschranke (4, 4a) mit einer Lichtquelle (5, 5a) aufweist,
wobei die Lichtquelle (5, 5a) an einem Wandabschnitt (6, 6a) der fluidführenden Komponente (1, 1a) angeordnet und zum Aussenden eines Lichtstrahls (S) eingerichtet ist,
wobei der Lichtstrahl (S) die in der fluidführenden Komponente (1, 1a) erstreckte Bewegungsbahn (B) des Stellelements (2, 2a) kreuzt,
und wobei der Lichtstrahl (S) in der ersten Stellung des Stellelements (2, 2a) ununterbrochen und in der zweiten Stellung des Stellelements (2, 2a) mittels des Stellelements (2, 2a) unterbrochen ist.

2. Vorrichtung (100) nach Anspruch 1, **dadurch gekennzeichnet, dass** der Wandabschnitt (6) für den Lichtstrahl (S) der Lichtquelle (5) transluzente Eigenschaften (T) aufweist, wobei die Lichtquelle (5) auf einer Außenseite (61) des Wandabschnitts (6) angeordnet ist, und wobei die Bewegungsbahn (B) des Stellelements (2) entlang einer der Außenseite (61) gegenüberliegenden Innenseite (62) des Wandabschnitts (6) erstreckt ist.

3. Vorrichtung (100) nach Anspruch 1, **dadurch gekennzeichnet, dass** der Wandabschnitt (6a) eine Apertur (64a) aufweist, welche quer zu der Bewegungsbahn (B) zwischen einer Innenseite (62a), entlang welcher die Bewegungsbahn (B) des Stellelements (2a) erstreckt ist, und einer der Innenseite (62a) gegenüberliegenden Außenseite (61a) des Wandabschnitts (6a) durchgängig erstreckt ist, wobei die Lichtquelle (5a) in die Apertur (64a) eingesetzt ist.

4. Vorrichtung (100) nach einem der vorhergehenden Ansprüche, **dadurch gekennzeichnet, dass** die Bewegungsbahn (B) und der Fluidleitungspfad (F) im Bereich des Wandabschnitts (6, 6a) parallel sind.

5. Vorrichtung (100) nach einem der vorhergehenden Ansprüche, **dadurch gekennzeichnet, dass** der Wandabschnitt (6, 6a) eine Lagerfläche (63, 63a) aufweist, an welcher das Stellelement (2, 2a) entlang der Bewegungsbahn (B) gleitbeweglich gelagert ist.

6. Vorrichtung (100) nach einem der vorhergehenden Ansprüche, **dadurch gekennzeichnet, dass** der Wandabschnitt (6, 6a) ein Abschnitt eines Gehäuses (7, 7a) der fluidführenden Komponente (1, 1a) ist, wobei der Fluidleitungspfad (F) wenigstens abschnittsweise durch das Gehäuse (7, 7a) erstreckt ist und das Stellelement (2, 2a) in dem Gehäuse (7, 7a) beweglich gelagert ist.

7. Vorrichtung (100) nach einem der vorhergehenden Ansprüche, **dadurch gekennzeichnet, dass** die fluidführende Komponente (1, 1a) ein Ventil (10, 10a), insbesondere ein Rückschlagventil, ist und das Stellelement (2, 2a) ein Ventilkörper (20, 20a) ist.

8. Vorrichtung (100) nach Anspruch 7, **dadurch gekennzeichnet, dass** der Wandabschnitt (6, 6a) ein Abschnitt eines Ventilgehäuses (70, 70a) des Ventils (10, 10a) ist, wobei das Ventilgehäuse (70, 70a) einen Einlass (71, 71a) zum Einleiten der Flüssigkeit und einen entlang des Fluidleitungspfads (F) fluidleitend mit dem Einlass (71, 71a) verbundenen Auslass (72, 72a) zum Ausleiten der Flüssigkeit aufweist, und wobei die Lichtschranke (4, 4a) entlang des Fluidleitungspfads (F) zwischen dem Einlass (71, 71a) und dem Auslass (72, 72a) an dem Wandabschnitt (6, 6a) des Ventilgehäuses (70, 70a) angeordnet ist.
